# NEW EUROPEAN PATENT SPECIFICATION

(11) **EP 2 313 104 B2**
(45) Date of publication and mention of the opposition decision: **26.08.2020**
(45) Mention of the grant of the patent: 01.11.2017
(21) Application number: 09735511.9
(22) Date of filing: 23.04.2009
(51) Int. Cl.: A61K 31/722, A61K 9/00, A61K 9/06, A61K 47/02, A61P 11/02, A61P 27/16, A61P 27/00

(54) **THIOLATED CHITOSAN GEL**
THIOLIERTE CHITOSAN GELE
GEL DE CHITOSANE THIOLÉ

(30) Priority: 24.04.2008 US 47590; 21.05.2008 US 55037
(43) Date of publication of application: 27.04.2011
(73) Proprietor: Medtronic, Inc, Minneapolis, MN 55432 (US)
(72) Inventor: GONZALEZ, Maria, Nieves, E-28923 Alcorcon Madrid (ES); TIJSMA, Edze, Jan, NL-6224 LD Maastricht (NL); SCHAFFHAUSEN, Nancy, NL-4907 PM Oosterhout (NL)
(74) Representative: August Debouzy
(86) International application number: PCT/US2009/041590
(87) International publication number: WO 2009/132226

(56) References cited:
- WO-A-03/020771
- WO-A-2007/071375
- WO-A-2008/008857
- WO-A-2008/097317
- BERNKOP-SCHNURCH ET AL: "Thiomers: A new generation of mucoadhesive polymers" ADVANCED DRUG DELIVERY REVIEWS, ELSEVIER BV, AMSTERDAM, NL, vol. 57, no. 11, 3 November 2005 (2005-11-03), pages 1569-1582, XP025284000 ISSN: 0169-409X [retrieved on 2005-11-03]
- BERNKOP-SCHNURCH A ET AL: "Thiomers: Preparation and in vitro evaluation of a mucoadhesive nanoparticulate drug delivery system" INTERNATIONAL JOURNAL OF PHARMACEUTICS, ELSEVIER BV, NL, vol. 317, no. 1, 6 July 2006 (2006-07-06), pages 76-81, XP025113028 ISSN: 0378-5173 [retrieved on 2006-07-06]
- ANONYMOUS: "Chitoflex Surgical"[Online] September 2007 (2007-09), XP002533528 Retrieved from the Internet: URL:http://www.hemcon.com/Portals/1/ChitoF lex%20-%20Surgical%20Product%20Overview%20 Brochure.pdf> [retrieved on 2009-06-23]
- KRAULAND et al.: "Viscoelastic Properties of a New in situ 2005 Gelling Thiolated Chitosan Conjugate", Drug Development And Industrial Pharmacy, vol. 31, 2005, pages 885-893,

## Description

### FIELD OF THE INVENTION

This invention relates to polysaccharides and to materials for use in the body.

### BACKGROUND

Certain polysaccharide materials have been used for surgical repair or drug delivery. Documents relating to such materials include U.S. Patent Nos. 6,514,522 (Domb) and 7,053,068 B2 (Prinz), U.S. Patent Application Publication Nos. US 2005/0176620 A1 (Prestwych et al.) and US 2005/0238702 A1 (Ishihara et al.), Canadian Patent Application No. 2 348 842 A1 (Bernkop-Schnürch), Published PCT Application Nos. WO 98/31712 A2 (B.F. Goodrich Co.), WO 01/00246 A2 (Bentley et al.) and WO 03/020771 A1 (Mucobiomer Biotechnologische Forschungs-und Entwicklungs GmbH), Mi et al., Synthesis and Characterization of a Novel Chitosan-Based Network Prepared Using Naturally-Occurring Crosslinker, J Polym Sci, Part A: Polym Chem, 38, 2804-2814 (2000), Mi et al., Synthesis and characterization of biodegradable TPP/genipin co-crosslinked chitosan gel beads, Polymer, 44, 6521-30 (2003), Roldo et al., Mucoadhesive thiolated chitosans as platforms for oral controlled drug delivery: synthesis and in vitro evaluation, European Journal of Pharmaceutics and Biopharmaceutics, 57, 115-121 (2004), Krauland et al., Viscoelastic Properties of a New in situ Gelling Thiolated Chitosan Conjugate, Drug Development And Industrial Pharmacy, 31, 885-893 (2005), Bernkop-Schnürch, Thiomers: A new generation of mucoadhesive polymers, Advanced Drug Delivery Reviews, 57, 1569-1582 (2005), Bernkop-Schnürch et al., Thiomers: Preparation and in vitro evaluation of a mucoadhesive nanoparticulate drug delivery system, International journal of Pharmaceutics, 317, 76-81 (2006) and Weng et al., Rheological Characterization of in Situ Crosslinkable Hydrogels Formulated from Oxidized Dextran and N-Carboxyethyl Chitosan, Biomacromolecules, 8, 1109-1115 (2007).

### SUMMARY OF THE INVENTION

Thiolated chitosans are capable of undergoing self-crosslinking reactions to form gels. The reported gelation times are 30 minutes or more and sometimes are expressed in hours. If used in some parts of the body, ample portions of the ungelled material might drain from the site or be carried away by ciliation or other natural defenses before gelation occurs.

External crosslinkers have been added to some chitosan materials to form nanoparticle beads or tablets for drug delivery. Beads and tablets, while useful for some applications, would not provide a protective sealing layer on mucosal tissue.

By combining a thiolated chitosan with an appropriate amount of one or both of an oxidizing agent and external crosslinking agent, the rate at which gelation occurs may be significantly increased. If the resulting mixture is spread into a layer atop a tissue or structure and if the amount of oxidizing agent or external crosslinking agent is not excessive, a protective gel layer can form. The protective gel layer may assist in returning an injured, inflamed or surgically repaired surface to a normal state, e.g., through one or more healing mechanisms such as modulation of an inflammatory response, phagocytosis, mucosal remodeling, reciliation or other full or partial restoration of normal function. The present invention provides in one aspect a method for treating a bodily tissue or structure, which method comprises:
a) applying thereto a fluid layer containing a mixture of thiolated chitosan and an oxidizing agent or external crosslinking agent, and
b) allowing the mixture to form a protective gel layer *in situ.*

The invention provides in another aspect a two-part composition for protecting a bodily tissue or structure, the first part comprising thiolated chitosan and the second part comprising sufficient oxidizing agent or external crosslinking agent so that when the two parts are mixed and coated onto the tissue or structure the mixture will form a protective gel layer in less than 30 minutes.

The invention provides in yet another aspect a fluid layer atop a bodily tissue or structure, the layer comprising thiolated chitosan and sufficient oxidizing agent or external crosslinking agent so that a protective gel layer will form *in situ* in less than 30 minutes.

The disclosed protective gel layer desirably is inherently antimicrobial without addition of a separate antimicrobial agent. The disclosed two-part composition desirably is packaged in a multicomponent spray dispenser, a multiple-barrel syringe or an ampoule with a rupturable or removable septum between the first and second parts. The disclosed method, composition and layer are especially useful for treating mucosal tissues in the ears, nose or throat and openings, recesses, passageways or joints in the limbs or spinal column.

The disclosed methods, compositions and fluid layers are not and do not utilize implants or implantable devices.

### BRIEF DESCRIPTION OF THE DRAWING

**Fig. 1** is a schematic view showing the disclosed method;
**Fig. 2** is a perspective view of a dispensing instrument which may be used in the disclosed method;
**Fig. 3** is a graph showing the gelation behavior and rheological properties of various thiolated chitosan solutions;
**Fig. 4** is a graph showing the viscosity of various chitosan solutions;
**Fig. 5** is a graph comparing the antimicrobial properties of an *in situ* crosslinked thiolated chitosan gel layer to a trypticase soy broth control; and
**Fig. 6** is a graph showing degradation of various thiolated chitosan gel layers.

Like reference symbols in the various figures of the drawing indicate like elements. The elements in the drawing are not to scale.

### DETAILED DESCRIPTION

The following detailed description describes certain embodiments and is not to be taken in a limiting sense. All weights, amounts and ratios herein are by weight, unless otherwise specifically noted. The terms shown below have the following meanings:

The term "adhesion" refers to the sticking together of a body structure or prosthetic material to tissue, to the sticking together of tissue to tissue with which it is in intimate contact for extended periods, or to the formation of tissue that connects body structures, prosthetic materials or tissues to one another across a normally open space.

The term "antimicrobial" refers to an ability to cause greater than a 90% numeric reduction (viz., at least a 1-log order reduction) in a population of one or more of *Staphylococcus aureus, Pseudomonas aeruginosa, Streptococcus pneumonia, Haemophilus influenzae* or *Moraxella catarrhalis.*

The terms "attached" and "adhered" when used in reference to a bacterial biofilm and a surface mean that the biofilm is established on and at least partially coats or covers the surface, and has some resistance to removal from the surface. As the nature of this relationship is complex and poorly understood, no particular mechanism of attachment or adherence is intended by such usage.

The term "bacterial biofilm" means a community of bacteria attached to a surface, with the organisms in the community being contained within an extracellular polysaccharide (EPS) matrix produced by the bacteria.

The term "biocompatible" when used in reference to a substance means that the substance presents no significant deleterious or untoward effects upon the body.

The term "biodegradable" when used in reference to a substance means that the substance will degrade or erode *in vivo* to form smaller chemical or physical species. Such degradation process may be enzymatic, chemical or physical.

The term "bioresorbable" when used in reference to a substance means that the substance is capable of being absorbed by the body.

The term "cohesive" when used in reference to a liquid or gel means that the liquid or gel when placed on a level surface will tend to (but need not in all cases) stick to itself and form a unitary mass.

The term "comminuted" when used in reference to a particulate material means that the particles have been fractured and reduced in size by cutting, grinding, pulverizing, triturating or other particle fracturing process employing externally-applied force.

The term "conformal" when used in reference to a composition applied to tissue or other body structure means that the composition can form a substantially continuous layer over an area to which the composition has been applied.

The terms "detaching", "removing" and "disrupting" when used in reference to a bacterial biofilm attached or adhered to a surface mean that at least a significant amount of the biofilm initially present on the surface no longer is attached or adhered to the surface. No particular mechanism of detachment, removal or disruption is intended by such usage.

The term "fluid" when used in reference to a substance means that the substance is a liquid having a loss modulus (G") greater than its storage modulus (G') and a loss tangent (tan δ) greater than 1.

The term "gel" when used in reference to a substance means that the substance is deformable (viz., is not a solid), G" is less than G' and tan δ is less than 1.

The term "gelation" when used with respect to formation of a gel layer means the time at which G" equals G' and tan δ equals 1.

The term "hemostat" means a device or material which stops blood flow.

The term "hydrogel" when used in reference to a gel means that the gel is hydrophilic and contains water.

The term "hydrated" when used in reference to a device or substance means that the device or substance contains uniformly distributed chemically-bound water. A "fully hydrated" device or substance is incapable of taking up additional water of hydration. A "partially hydrated" device or substance is capable of taking up additional water of hydration.

The term "implant" means a sterile, solid preparation of a size and shape suitable for parenteral implantation and release of one or more active substances over a period of time.

The term "implantable device" means a solid device intended to be totally introduced into the human body or to replace an epithelial surface or the surface of the eye via a surgical procedure, and which is intended to remain in place after the procedure. Any solid device intended to be partially introduced into the human body through a surgical procedure and intended to remain in place for at least 30 days after the procedure will also be considered an implantable device.

The term "inner ear" means the semicircular canals and cochlea.

The term "middle ear" means the region defined by the tympanic membrane, interior structures such as the ossicular chain, the surrounding lining and bordering structures such as the mastoid.

The term "mucoadhesive" when used in reference to a device or substance means that the device or substance will adhere to the mucus covering epithelia.

The term "nasal or sinus cavities" refers to the various tissues defining the normally air-filled passages and chambers within the nose and sinus including but not limited to the nostrils or nares, the nasal concha or turbinates, the frontal, ethmoid, sphenoid and maxillary sinuses, the sinus ostia and the nasopharnyx.

The term "polysaccharide" includes derivatives of polysaccharides and modified polysaccharides, as well as derivatives of individual polysaccharide species and modified individual polysaccharide species. For example, the term "carboxymethylcellulose" includes carboxymethylcellulose derivatives and modified carboxymethylcelluloses, the term "chitosan" includes chitosan derivatives and modified chitosans, and the term "starch" includes starch derivatives and modified starches.

The term "protective" when used in reference to a layer of a composition atop tissue or other body structure means that the layer may assist in returning an injured, inflamed or surgically repaired tissue surface to a normal state, e.g., through one or more healing mechanisms such as modulation of an inflammatory response, phagocytosis, mucosal remodeling, reciliation or other full or partial restoration of normal function.

The term "residence time" when used in reference to a protective gel layer atop tissue or other body structure means the time period during which the gel layer or portion thereof remains in place *in vivo* under gross observation.

The term "solvating" means to form a solution or dispersion containing a solvent or other carrier within which a solute is dissolved or suspended.

The term "substantially collagen-free" means containing a sufficiently low amount of collagen so as not to pose a potential risk of transmission of or infection with bovine spongiform encephalopathy (BSE) or variant Creutzfeldt-Jakob disease (vCJD).

The term "thin" when used in reference to a protective layer atop tissue or other body structure means having an average thickness less than about two millimeters.

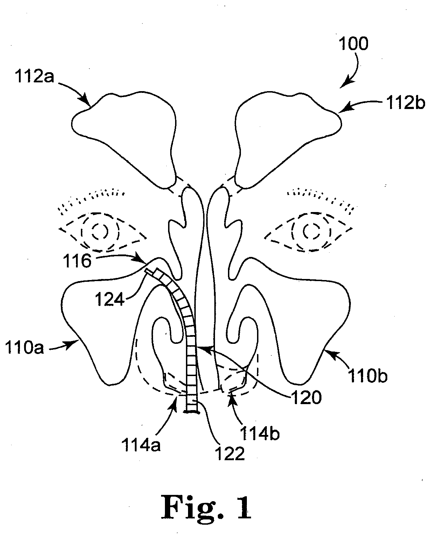

Referring to **Fig. 1**, the disclosed method may be performed for example in the nasal or sinus cavities **100** of a patient, including the maxillary sinuses **110a**, **110b** and frontal sinuses **112a**, **112b,** which may be accessed through nares **114a**, **114b.** It should be noted that external features of the patient, including nares **114a**, **114b**, are shown in dashed lines. When the patient suffers for example from chronic rhinosinusitis, one or more treatment sites such as treatment site **116** associated with a surface of maxillary sinus **110a** may be medically or if need be surgically addressed. Treatment site **116** includes ciliated epithelium of maxillary sinus **110a** and may include an associated layer of bacteria inhabiting an associated biofilm (not shown in **Fig. 1**). The treatment site need not be natural tissue and may instead be an artificial structure (not shown in **Fig. 1**) such as a sinus packing or stent which may also be covered at least in part with a layer of bacterial biofilm. If present, the biofilm may be removed using a solvating system (for example, the solvating system described in U.S. Patent Application Publication No. US 2007/0264310 A1) which may be applied to treatment site **116** using an introducer **120** with an articulatable delivery tube **122** containing an irrigation duct (hidden in **Fig. 1**) through which the solvating system may flow to a nozzle **124** at the distal end of introducer **122** and thence to the treatment site. The solvating system and residues of the biofilm may be removed from the treatment site via an aspiration duct (hidden in **Fig. 1**). The disclosed thiolated chitosan composition may likewise be applied at the treatment site using the same or a different irrigation duct in introducer **120**. Those skilled in the art will appreciate that the disclosed thiolated chitosan composition (and if used, the solvating system) may be applied to the treatment site using other methods or devices. Exemplary other methods include power spray or other spray application, lavage, misting, mopping, wicking, dripping and trephination and exemplary other devices include spray nozzles (e.g., single component or multiple component spraying nozzles) and syringes (e.g., single barrel or multiple barrel glass or plastic syringes and bulb syringes). The treatment method may also be performed in other parts of the body. The treatment method has particular utility in non-vascular applications, including treatment of tissues (e.g., mucosal tissues) or structures in or near the ears, throat, limbs or spinal column.

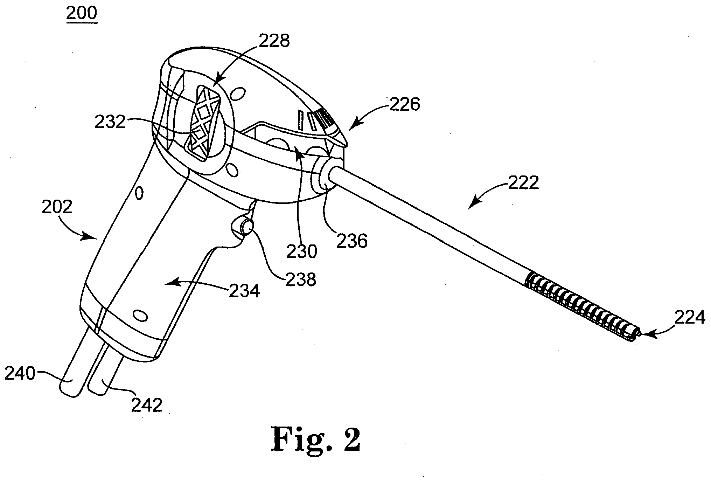

**Fig. 2** shows an exemplary instrument **200** which may be used in the disclosed treatment method. Instrument **200** includes a handle **202** and an introducer **222** whose distal end **224** (referenced generally) includes a spray nozzle, irrigation and aspiration ducts (not separately numbered in **Fig. 2**). Instrument **200** can optionally further include a first actuator assembly **226** (referenced generally) and a second actuator assembly **228** (referenced generally). A control wheel **230** in first actuator assembly **226** may be operable by a user to effectuate bending of the introducer **222**, and a control wheel **232** in second actuator assembly **228** may be operable by a user to effectuate movement or rotation relative to introducer **222** of liquid sprayed from distal end **224** of introducer **222**. Handle **202** serves generally as a housing for various other components of instrument **200** and as a support for introducer **222**. Handle **202** may have a pistol grip-like shape, defining a grip portion **234** and a nose **236**. Grip portion **234** is sized and shaped for grasping by a user's hand, whereas nose **236** is adapted for connection to introducer **222**. Trigger **238** and an associated sensor and valve (not shown in **Fig. 2**) may be used to control the flow of the disclosed rehydrated gel (and if used, the disclosed solvating system) through irrigation tubing **240** and thence through the spray nozzle in distal end **224** and onto the desired treatment site. Trigger **238** may be provided with a multidirectional range of motion and associated with one or more additional sensors and valves (not shown in **Fig. 2**) to control removal from a treatment site of the solvating system, biofilm residue and other debris through the aspiration duct in distal end **224** and thence to aspiration tubing **242**. Trigger **238** may also be used to control the flow of the disclosed rehydrated gel through a separate lumen in irrigation tubing **240** and thence through the spray nozzle in distal end **224** and onto the desired treatment site.

The applied thiolated chitosan composition may fill the treatment site (e.g., a nasal or sinus cavity, or an opening, recess, passageway or joint in a portion of the limbs or spinal column) in which case the disclosed layer may be very thick and not exposed to air or other nearby gases, and with differing thicknesses throughout the layer. The thiolated chitosan composition may also be applied as a film or other conformal coating in which case the disclosed layer may be relatively thin and exposed to air or other nearby gases, and with a substantially uniform thickness throughout the layer. After gelation the protective gel layer may be viscous, elastic or viscoelastic. The protective gel layer desirably adheres to mucosal or other natural tissues (e.g., cartilage or bone) at the treatment site and resists detachment or other disruption until natural degradation or resorption of the gel layer takes place, e.g., after a residence time in vivo of from one day to a few (e.g., 2, 3 or 4) days, weeks or months. Meanwhile bacterial recolonization or reinfection may be significantly reduced or prevented, and improved healing and reciliation may take place. The protective gel layer may provide various therapeutic advantages including but not limited to bacterial adhesion repellence, anti-infective properties, local immune modulation, tissue protection, reduction or elimination of pain or bleeding, reduction in inflammation, optimization of environment for ciliary regrowth, reduction in adhesions to critical anatomy, and the like. These advantages may arise due to a variety of mechanisms including a) killing bacteria, b) inhibiting bacterial colonization, c) inhibiting the adherence of bacteria to tissue, d) reducing tissue morbidity or abscess formation, e) reducing or preventing disease recurrence (for example, specifically reducing the chronic inflammation related to bacterial toxin and EPS), f) coating and protecting tissue during healing, such as by maintenance of a moist wound which promotes platelet aggregation, or by closure of a dry wound without excessive scabrous formation, g) hemostasis, h) optimizing the environment for reciliation of the mucosa, i) speeding the growth or regrowth of cilia and j) delivering therapeutic agent(s) to the treatment site. Desirably the protective gel layer will adhere to a portion of the mucosa while leaving the cilia in unadhered portions free to undergo natural rhythmic cilia motion (viz., cilia beating), will if desired also deliver antimicrobial agents or additional therapeutic agents, and desirably will discourage or prevent bacteria from adhering to the treatment site.

Exemplary thiolated chitosans may be obtained from a variety of commercial sources including ThioMatrix Forschungs Beratungs GmbH and Mucobiomer Biotechnologische Forschungs-und Entwicklungs GmbH or prepared by reaction of chitosan with a suitable thiolated reactant, e.g., as described in the above-mentioned Published PCT Application No. WO 03/020771 A1 or in the above-mentioned Roldo et al., Krauland et al., Bernkop-Schnürch and Bernkop-Schnürch et al. papers. Exemplary thiolated reactants include thioglycolic acid, cysteine, thiobutylamidine, glutathione and thioethylamidine.

The thiolated chitosan may have a variety of number average molecular weights, e.g., about 5 to about 2000 kDa, about 10 to about 500 kDa, or about 10 to about 100 kDa. The thiolated chitosan may for example be an ultralow molecular weight material having a number average molecular weight less than about 50 kDa, a low molecular weight material having a number average molecular weight of about 50 to about 200 kDa, a medium molecular weight material having a number average molecular weight of about 200 to about 500 kDa or a high molecular weight material having a number average molecular weight greater than about 500 kDa. The thiolated chitosan typically will contain a plurality of pendant thiol groups connected, via a short chain structure including one or more carbonyl or amine sites, to an amine group on the chitosan ring, and may for example have a degree of thiolation (expressed in µmol SH/g) of about 50 to about 1000, or about 400 to about 1000.

The thiolated chitosan desirably is obtained in dry particulate form, for example, as free-flowing granules whose average particle diameter is less than about 1 mm, less than about 100 µm, about 1, to about 80 µm, or less than 1 µm. The thiolated chitosan preferably is packaged and shipped to a user in such dry particulate form so as to reduce degradation of the thiolated chitosan during prolonged storage. The thiolated chitosan solution may be formed for example by dissolving the thiolated chitosan in water or another suitable solvent just prior to use. Recommended thiolated chitosan amounts will depend on the thiolated chitosan molecular weight, and may for example be about 1 to about 20 %, about 1 to about 10 % or about 1 to about 5 % of the resulting solution. U.S. Published Application No. US 2009/0285897 A1 describes a preferred technique for rehydrating a thiolated chitosan, by dispersing free-flowing thiolated chitosan particles in a biocompatible water-miscible polar dispersant, and combining the dispersion with sufficient aqueous solvent for the particles to convert them to a cohesive hydrogel. The thiolated chitosan may be comminuted but desirably is non-comminuted.

The oxidizing agent or external crosslinking agent (which each also may be in solution form) are meanwhile normally kept separate from the thiolated chitosan until just prior to use. Exemplary oxidizing agents which may be used include those listed at http://www.organic-chemistry.org/chemicals/oxidations, for example hydrogen peroxide, horseradish peroxidase, mushroom tyrosinase, oxygen, periodates, and mixtures thereof. Exemplary external crosslinking agents which may be used include low molecular weight aldehydes, such as genipin and glutaraldehyde, and polymeric aldehydes, such as oxidized polysaccharides. Mixtures containing both an oxidizing agent and an external crosslinking agent may be used if desired. Sufficient oxidizing agent or external crosslinking agent desirably are employed so that a protective gel layer will form in less than 30 minutes after mixing, and more preferably in less than 20 minutes, less than 10 minutes, less than 5 minutes or essentially immediately after mixing. For example, when using an oxidizing agent such as hydrogen peroxide, recommended amounts include about 0.01 to about 1 % or about 0.1 to about 1 % based on the weight of the final, thiolated chitosan-containing mixture. When using an external crosslinker such as genipin, recommended amounts include about 0.1 to about 10 % or about 0.25 to about 1 % of the mixture. Excessive amounts of the oxidizing agent or external crosslinker may undesirably reduce the viscosity of the mixture or inflame or otherwise irritate sensitive tissues.

The disclosed thiolated chitosan compositions desirably are substantially collagen-free. Preferably the thiolated chitosan compositions are sufficiently free of collagen (e.g., containing no collagen at all) so as to be saleable worldwide for use without restriction in humans.

The disclosed thiolated chitosan compositions may optionally include a variety of other ingredients. These other ingredients may be disposed before mixing in the first part, second part or both parts of a two-part composition. Exemplary other ingredients include water and other solvents (e.g., alcohols), acids, bases, buffering agents, antimicrobial agents, therapeutic agents and other adjuvants. An acid, base or buffering agent may for example maintain the composition at an appropriate pH for contacting human tissue, e.g., a pH greater than 5, a near-neutral pH, or a pH less than 8.5. Exemplary buffering agents include barbitone sodium, glycinamide, glycine, potassium chloride, potassium phosphate, potassium hydrogen phthalate, sodium acetate, sodium citrate, sodium phosphate and their conjugate acids.

The disclosed thiolated chitosan compositions desirably are inherently antimicrobial without requiring addition of a separate antimicrobial agent. A separate antimicrobial agent may however be employed if desired. A useful list of such antimicrobial agents may be found, for example, in the above-mentioned U.S. Patent Application Publication No. US 2007/0264310 A1.

Exemplary therapeutic agents which may be employed in the disclosed thiolated chitosan compositions include any material suitable for use at the intended treatment site including analgesics, anti-cholinergics, anti-fungal agents, antihistamines, steroidal or non-steroidal anti-inflammatory agents, anti-parasitic agents, antiviral agents, biostatic compositions, chemotherapeutic/antineoplastic agents, cytokines, decongestants, hemostatic agents (e.g., thrombin), immunosuppressors, mucolytics, nucleic acids, peptides, proteins, steroids, vasoconstrictors, vitamins, mixtures thereof, and other therapeutic materials that will be known to those skilled in the art. A useful list of such therapeutic agents may be found, for example, in the above-mentioned U.S. Patent Application Publication No. US 2007/0264310 A1.

Other adjuvants that may be included in the disclosed thiolated chitosan compositions include dyes, pigments or other colorants (e.g., FD & C Red No. 3, FD & C Red No. 20, FD & C Yellow No. 6, FD & C Blue No.2, D & C Green No. 5, D & C Orange No. 4, D & C Red No. 8, caramel, titanium dioxide, fruit or vegetable colorants such as beet powder or beta-carotene, turmeric, paprika and other materials that will be known to those skilled in the art); indicators; flavoring or sweetening agents including but not limited to anise oil, cherry, cinnamon oil, citrus oil (e.g., lemon, lime or orange oil), cocoa, eucalyptus, herbal aromatics (e.g., clove oil, sage oil or cassia oil), lactose, maltose, menthol, peppermint oil, saccharine, sodium cyclamate, spearmint oil, sorbitol, sucrose, vanillin, wintergreen oil, xylitol and mixtures thereof; antioxidants; antifoam agents; and rheology modifiers including thickeners and thixotropes. The disclosed thiolated chitosan compositions desirably do not contain ingredients which might potentially harm mucosal tissues or structures, e.g., tissues in the nasal or sinus cavities.

In those instances where it is desirable to remove water from tissue, e.g., to remove fluid from polyps or edematous tissue, a hyperosmolar agent may be employed in the disclosed thiolated chitosan compositions. Exemplary hyperosmolar agents include furosemide, sodium chloride gel and other salt preparations that draw water from tissue or substances which directly or indirectly change the osmolar content of the mucous layer. Where sustained release or delayed release of a therapeutic agent is desirable, a release agent modifier may also be included.

The disclosed composition typically will be subjected to sterilization and placed in suitable sealed packaging (for example, a multicomponent syringe, a vial or vials, or a multi-chamber pouch made of suitable materials) prior to shipment to an end user. Additional property customization may be carried out by using a sterilization procedure such as gamma radiation or electron beam (E-Beam) processing to cause controlled chain scission. Cold ionizing radiation sterilization (e.g., cold E-Beam sterilization) may be employed to limit the degree of chain scission, as discussed in copending PCT Application No. (Attorney Docket Nos. P0035142.00 and 151-P-35142WO01), filed even date herewith. Whether or not sterilized, the first part containing the thiolated chitosan normally will be kept separate from the second part containing the oxidizing agent or external crosslinking agent until just prior to use.

The disclosed thiolated chitosan compositions may desirably be used as a part of a multi-step treatment regimen which disrupts a bacterial biofilm and discourages its return. For example, a series of steps that may be broadly classified as Cleansing/Disrupting, Killing, Aerating, Protecting/Coating, and Healing may be carried out. The Cleansing/Disrupting step may be carried out by administering a solvating system as discussed above in connection with **Fig. 1** and **Fig. 2****.** The Killing step may be carried out by applying a suitable antimicrobial agent to the treatment site. This may for example be accomplished by including an antimicrobial agent in the solvating system, as a separately-applied composition, or in both the solvating system and in a separately-applied composition. An antimicrobial agent may also be applied or administered post operatively. The Aerating step may be carried out by providing air passageways or improving air passageways to the treated tissues by opening occluded or partially occluded passages, e.g., the sinuses or sinus ostia for nasal applications. This may for example be accomplished by surgically removing obstructive tissue structures or by manually displacing such structures. The Protecting/Coating step may be carried out by coating at least part of the thus-treated tissue with the disclosed thiolated chitosan composition. The Healing step may be carried out by allowing the cleansed, protected and sealed tissue surface to undergo a return to a normal state, e.g., through one or more healing mechanisms such as modulation of an inflammatory response, phagocytosis, mucosal remodeling, reciliation or full or partial restoration of normal function. The multi-step treatment regimen may include or be followed by a Clearing step in which the disclosed thiolated chitosan composition is sufficiently biodegradable or bioresorbable to disappear from the treatment site in a desired time period, e.g., more than 1 day, more than 3 days, or about 4 to 7 days, and desirably without shedding large solid chunks. The disclosed method may advantageously be accomplished without requiring surgery, for example by applying and removing the optional solvating system and by applying the disclosed thiolated chitosan composition through normal aspiration/suction techniques or by simple flushing of affected tissue. A comparable series of steps may be performed in a multi-step treatment regimen in a portion of the middle or inner ear. Further details regarding such a regimen may be found in U.S. Patent Application Publication No. US 2007/0264310 A1.

The invention is further illustrated in the following non-limiting examples.

### Example 1

Thiolated chitosan materials were obtained in lyophilized form from ThioMatrix Forschungs Beratungs GmbH (Innsbruck, Austria). Thioglycolic acid-modified (TGA-modified) chitosan materials were prepared as described in Kast et al., Thiolated polymers-thiomers: development and in vitro evaluation of chitosan-thioglycolic acid conjugates, Biomaterials, 22, 2345-2352 (2001). Thiobutylamidine-modified (TBA-modified) materials were prepared as described in the above-mentioned Roldo et al. paper. Characteristics of the chitosan materials are shown below in Table 1:

**Table 1**

| **Thiolated Chitosan Materials** | | |
|---|---|---|
| **Material¹** | **Type of thiolation** | **Degree of thiolation (µmol SH/g)** |
| ULCS-TGA Low | Thioglycolic acid | 59 |
| HCS-TGA Low | Thioglycolic acid | 57 |
| ULCS-TBA Low | Thiobutylamidine | 72 |
| HCS-TBA Low | Thiobutylamidine | 54 |
| ULCS-TGA High | Thioglycolic acid | 310 |
| HCS-TGA High | Thioglycolic acid | 265 |
| ULCS-TBA High | Thiobutylamidine | 425 |
| HCS-TBA High | Thiobutylamidine | 226 |
| ¹ ULCS = Ultra low molecular weight chitosan; HCS = High molecular weight chitosan | | |

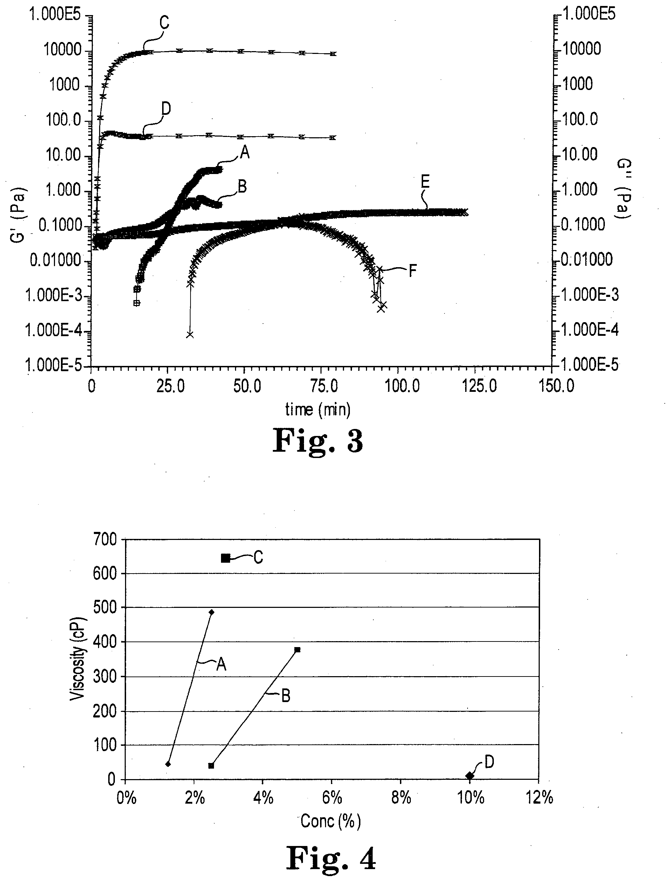

**In-situ gelation of thiolated chitosan.** 150 mg of ULCS-TBA High was dissolved in 4 mL deionized water. The solution was adjusted to pH 6.0 using 0.5 M NaOH and diluted to 9 mL followed by the addition of 1 mL of a 1 % (m/m) hydrogen peroxide solution. The resulting formulation contained 1.5 % thiolated chitosan and formed a gel in less than 30 minutes as determined using rheology measurements (G' ≥ G" see Curve **A** for G' and Curve **B** for G" in **Fig. 3**). A formulation containing 500 mg ULCS-TBA High (5 %) and hydrogen peroxide gelled in 5 minutes (see Curve **C** for G' and Curve **D** for G" in **Fig. 3**), whereas a formulation containing 500 mg ULCS-TBA High (5 %) and no hydrogen peroxide required 60 minutes to gel (see Curve **E** for G' and Curve **F** for G" in **Fig. 3**). The formulation containing 500 mg ULCS-TBA high (5 %) and hydrogen peroxide exhibited very favorable rheological properties including a low initial G' value (less than 0.1 Pa) at the start of mixing and much greater G" value (more than 8,000 Pa) within about 10 minutes.

Additional gelation experiments were performed using formulations containing 1.5 % HCS-TBA Low or 1.5 % HCS-TBA High in the presence of the aldehyde crosslinker genipin. In each case, gels were formed in 5-10 minutes with G' values of about 100-200 Pa.

### Example 2

### Sprayability

**Viscosity of thiolated chitosan solutions.** The viscosities of two non-thiolated chitosan solutions respectively made using high and low molecular weight chitosan salt (Curves **A** and **B** in **Fig. 4**) and two thiolated chitosan solutions respectively made using 2.5 % HCS-TGA Low and 10 % ULCS-TGA Low (datapoints **C** and **D** in **Fig. 4**) were determined in deionized water at 25 °C. The HCS-TGA Low solution had a relatively high viscosity of about 650 cP whereas the ULCS-TGA Low solution had a very low viscosity of about 10 cP. From these results and spraying experiments conducted using other chitosan solutions it appeared that the ULCS-TGA Low solution would readily be sprayable at concentrations at and likely well above 10 %.

### Example 3

### Antimicrobial Properties

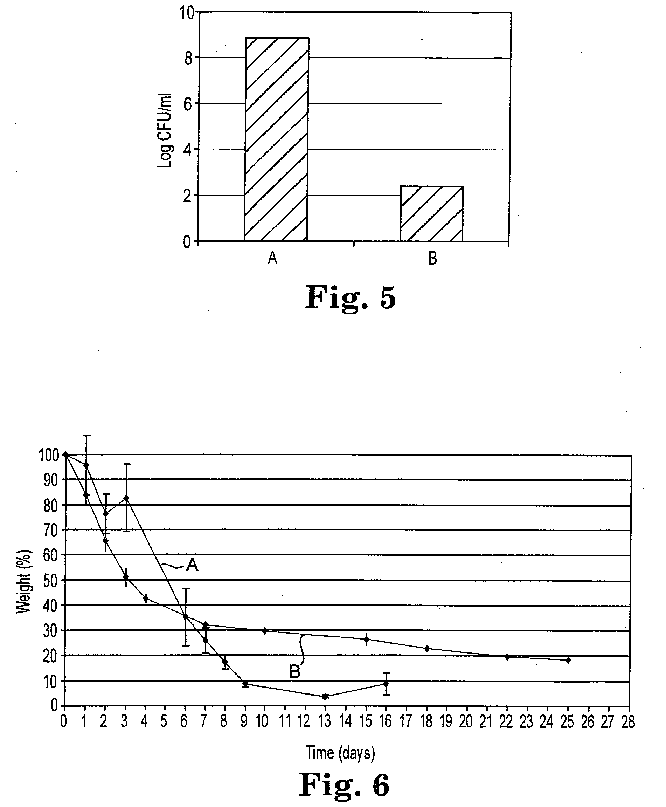

**Antimicrobial properties of thiolated chitosan gels.** The antimicrobial activity of gels made using the 1.5 and 5 % ULCS-TBA High formulations and 0.1 % hydrogen peroxide was determined versus *S. Aureus.* The thiolated chitosan gel formulations were placed in duplicate under sterile conditions directly into a 24-well polystyrene tissue culture plate. Each well was incubated with 1 mL (500,000 colony forming units) of a bacterial suspension of *S. Aureus* (ATCC 25923). Positive controls were incubated with 1 ml of trypticase soy broth (TSB). After 6 hours incubation at 37 °C, the media was transferred in new tubes and serial ten-fold dilutions were performed. Ten µL aliquots from the appropriate dilution were plated in triplicate on trypticase soy agar plates using the dilution track method (Jett B.D. et al., Biotechniques, 23, 648-650 (1997). The plates were incubated at 37 °C for 24 hours and Colony Forming Units (CFU) were counted. As shown in **Fig. 5**, the 5 % gel (bar **B**) showed complete (greater than 6 log reduction) killing of the bacteria vs. a trypticase soy broth (TSB) control (bar A). Similar results (a 6 log reduction) were obtained using the 1.5 % gel.

### Example 4

### Degradation

**Degradation of thiolated CS gels.** The degradation behavior of gels made using 1.5 % (see Curve **A** in **Fig. 6**) and 5 % (see Curve **B** in **Fig. 6**) ULCS-TBA High and 0.1 % hydrogen peroxide was determined by placing the gels in phosphate buffered saline (PBS) at pH 7.4 in the presence of lysozyme (1 mg/mL). The weight loss was determined at various time points up to 28 days. As shown in **Fig. 6**, significant weight loss was observed. After 28 days the samples had not fully degraded.

The results in Examples 1-4 show that thiolated chitosan may be used to prepare injectable or sprayable formulations which quickly gel *in situ* to form inherently antimicrobial protective layers.

## Claims

1. A two-part composition suitable for use in protecting a mucosal tissue in the ear, nose or throat, the first part comprising thiolated chitosan and the second part comprising sufficient oxidizing agent or external crosslinking agent so that when the two parts are mixed and coated onto the mucosal tissue the mixture will form a protective gel layer in less than 30 minutes.

2. A composition according to claim 1 wherein the first and second parts are each packaged in a barrel of a multiple-barrel syringe.

3. A composition according to any one of claims 1 to 2 wherein the first part is a free-flowing dry powder.

4. A composition according to any one of claims 1 to 2 wherein the first and second part are each an aqueous solution.

5. A composition according to any one of claims 1 to 4 wherein the second part contains hydrogen peroxide.

6. A composition according to any one of claims 1 to 5 wherein the composition contains 1 to 20 wt. % thiolated chitosan and 0.01 to 1 wt. % oxidizing agent.

7. A composition according to any one of claims 1 to 4 wherein the second part contains genipin.

8. A composition according to one any of claims 1 to 4 or 7 wherein the composition contains 1 to 20 wt. % thiolated chitosan and 0.1 to 10 wt. % external crosslinking agent.

9. A composition according to any one of claims 1 to 8 wherein when the two parts are mixed a gel layer will form in less than 10 minutes.

10. A fluid layer atop mucosal tissue in the ears, nose or throat, the layer comprising a mixture of a two-part composition comprising thiolated chitosan and an oxidizing agent or external crosslinking agent as defined in any one of claims 1 to 9.

11. A fluid layer according to claim 10 wherein the tissue comprises nasal tissue, sinus tissue or middle or inner ear tissue.

12. A fluid layer for use in a method for returning an injured, inflamed or surgically repaired mucosal tissue surface in the ear, nose or throat to a normal state, which method comprises:
a) applying to said tissue surface a fluid layer containing a mixture of a two-part composition comprising thiolated chitosan and an oxidizing agent or external crosslinking agent as defined in any one of claims 1 to 9, and
b) allowing the mixture to form a protective gel layer in situ as a tissue sealant.

13. A fluid layer for use according to claim 12, wherein said fluid layer is applied to a nasal cavity, a sinus cavity or a middle or inner ear.

14. A fluid layer for use according to claim 12 or claim 13, wherein said fluid layer is applied by spraying, lavage, misting, mopping, wicking or dripping.

## Patentansprüche

1. Zweiteilige bzw. Zwei-Bestandteile-Zusammensetzung, die geeignet zur Verwendung im Schützen eines Schleimhautgewebes in dem Ohr, der Nase oder dem Hals ist, wobei der erste Bestandteil thioliertes Chitosan umfasst und der zweite Bestandteil genügend Oxidationsmittel oder externen Vernetzer umfasst, so dass, wenn die zwei Bestandteile gemischt und auf das Schleimhautgewebe aufgetragen werden, das Gemisch eine schützende Gelschicht in weniger als 30 Minuten bildet.

2. Zusammensetzung nach Anspruch 1, wobei der erste und zweite Bestandteil jeweils in einem Zylinder einer Mehrzylinderspritze verpackt sind.

3. Zusammensetzung nach einem der Ansprüche **1** bis 2, wobei der erste Bestandteil ein fließfähiges trockenes Pulver ist.

4. Zusammensetzung nach einem der Ansprüche **1** bis 2, wobei der erste und zweite Bestandteil jeweils eine wässrige Lösung sind.

5. Zusammensetzung nach einem der Ansprüche **1** bis 4, wobei der zweite Bestandteil Wasserstoffperoxid enthält.

6. Zusammensetzung nach einem der Ansprüche **1** bis 5, wobei die Zusammensetzung **1** bis 20 Gewichts-% thioliertes Chitosan und 0,01 bis **1** Gewichts-% Oxidationsmittel enthält.

7. Zusammensetzung nach einem der Ansprüche **1** bis 6, wobei der zweite Bestandteil Genipin enthält.

8. Zusammensetzung nach einem der Ansprüche **1** bis 4 oder 7, wobei die Zusammensetzung **1** bis 20 Gewichts-% thioliertes Chitosan und 0,1 bis 10 Gewichts-% externen Vernetzer enthält.

9. Zusammensetzung nach einem der Ansprüche **1** bis 8, wobei, wenn die zwei Bestandteile gemischt werden, eine Gelschicht sich in weniger als 10 Minuten bildet.

10. Fluidschicht auf Schleimhautgewebe in den Ohren, der Nase oder dem Hals, wobei die Schicht ein Gemisch einer Zwei-Bestandteile-Zusammensetzung umfasst, die thioliertes Chitosan und ein Oxidationsmittel oder einen externen Vernetzer wie in einem der Ansprüche **1** bis 9 definiert umfasst.

11. Fluidschicht nach Anspruch 10, wobei das Gewebe nasales Gewebe, Nasennebenhöhlengewebe oder Mittel- oder Innenohrgewebe umfasst.

12. Fluidschicht zur Verwendung in einem Verfahren zum Zurückführen einer verletzten, entzündeten oder chirurgisch reparierten Schleimhautgewebeoberfläche in dem Ohr, der Nase oder dem Hals zu einem normalen Zustand, wobei das Verfahren umfasst:
a) Aufbringen einer Fluidschicht, die ein Gemisch einer Zwei-Bestandteile-Zusammensetzung umfasend thioliertes Chitosan und ein Oxidationsmittel oder einen externen Vernetzer wie in einem der Ansprüche **1** bis 9 definiert enthält, auf die Gewebeoberfläche; und
b) Zulassen, dass das Gemisch eine schützende Gelschicht in situ als ein Gewebeversiegelungsmittel bildet.

13. Fluidschicht zur Verwendung nach Anspruch 12, wobei die Fluidschicht auf einen nasalen Hohlraum, einen Nasennebenhöhlenhohlraum oder ein Mittel- oder Innenohr aufgebracht wird.

14. Fluidschicht zur Verwendung nach Anspruch 12 oder 13, wobei die Fluidschicht durch Sprühen, Spülung, Umneblung, Abtupfen, Dochtaufbringung bzw. Wieking oder Abtropfen aufgebracht wird.

## Revendications

1. Composition en deux parties adaptée pour être utilisée pour protéger un tissu muqueux dans l'oreille, le nez ou la gorge, la première partie comportant du chitosane thiolé et la seconde partie comportant un agent oxydant ou un agent de réticulation externe suffisant pour que, lorsque les deux parties sont mélangées et déposées sur le tissu muqueux, le mélange forme une couche de gel protectrice en moins de 30 minutes.

2. Composition selon la revendication 1, dans laquelle les première et seconde parties sont chacune emballées dans un cylindre d'une seringue à plusieurs cylindres.

3. Composition selon l'une quelconque des revendications 1 à 2, dans laquelle la première partie est une poudre sèche à écoulement libre.

4. Composition selon l'une quelconque des revendications 1 à 2, dans laquelle les première et seconde parties sont chacune une solution aqueuse.

5. Composition selon l'une quelconque des revendications 1 à 4, dans laquelle la seconde partie contient du peroxyde d'hydrogène.

6. Composition selon l'une quelconque des revendications 1 à 5, dans laquelle la composition contient 1 à 20 % en poids de chitosane thiolé et 0,01 à 1 % en poids d'agent oxydant.

7. Composition selon l'une quelconque des revendications 1 à 4, dans laquelle la seconde partie contient de la géni-pine.

8. Composition selon l'une quelconque des revendications 1 à 4 ou 7, dans laquelle la composition contient 1 à 20 % en poids de chitosane thiolé et 0,1 à 10% en poids d'agent de réticulation externe.

9. Composition selon l'une quelconque des revendications 1 à 8, dans laquelle lorsque les deux parties sont mélangées, une couche de gel se formera en moins de 10 minutes.

10. Couche fluide au-dessus d'un tissu muqueux dans les oreilles, le nez ou la gorge, la couche comportant un mélange d'une composition en deux parties comportant du chitosane thiolé et un agent oxydant ou un agent de réticulation externe tels que définis dans l'une quelconque des revendications 1 à 9.

11. Couche fluide selon la revendication 10, dans laquelle le tissu comporte un tissu nasal, un tissu sinusal ou un tissu d'oreille moyenne ou interne.

12. Couche fluide destinée à être utilisée dans un procédé pour ramener à un état normal une surface de tissu muqueux abîmée, enflammée ou réparée chirurgicalement dans l'oreille, le nez ou la gorge, lequel procédé comporte les étapes consistant à :
a) appliquer sur ladite surface de tissu une couche fluide contenant un mélange d'une composition en deux parties comportant du chitosane thiolé et un agent oxydant ou un agent de réticulation externe tels que définis dans l'une quelconque des revendications 1 à 9, et
b) permettre au mélange de former une couche de gel protectrice *in situ* faisant office d'agent d'étanchéité tissulaire.

13. Couche fluide pour une utilisation selon la revendication 12, dans laquelle ladite couche fluide est appliquée sur une cavité nasale, une cavité sinusale ou une oreille moyenne ou interne.

14. Couche fluide pour une utilisation selon la revendication 12 ou la revendication 13, dans laquelle ladite couche fluide est appliquée par pulvérisation, lavage, nébullisation, étalement, pénétration capillaire ou suintement.
